# EUROPÄISCHE PATENTSCHRIFT

(11) **EP 1 868 431 B1**
(45) Veröffentlichungstag und Bekanntmachung des Hinweises auf die Patenterteilung: **26.05.2010**
(21) Anmeldenummer: 06722793.4
(22) Anmeldetag: 13.04.2006
(51) Int. Cl.: A01N 25/22, A61L 2/18

(54) **DESINFEKTIONSMITTEL MIT KEIMABTÖTENDEN EIGENSCHAFTEN**
DISINFECTANT WITH GERM-KILLING PROPERTIES
AGENT DE DESINFECTION A PROPRIETES GERMICIDES

(30) Priorität: 14.04.2005 DE 102005017384
(43) Veröffentlichungstag der Anmeldung: 26.12.2007
(73) Patentinhaber: Ropimex R. Opel GmbH, 66538 Neunkirchen (DE); Sarastro GmbH, 66287 Göttelborn (DE)
(72) Erfinder: HANSELMANN, Rainer, G., 66119 Saarbrücken (DE); SCHIRRA, Hermann, 66111 Saarbrücken (DE)
(74) Vertreter: Wieske, Thilo
(86) Internationale Anmeldenummer: PCT/DE2006/000668
(87) Internationale Veröffentlichungsnummer: WO 2006/108410

(56) Entgegenhaltungen:
- WO-A-96/26719
- WO-A-2006/010667
- US-A- 4 205 096
- US-A1- 2002 037 370
- US-A1- 2002 150 724
- US-A1- 2003 118 624
- US-A1- 2003 147 925

## Beschreibung

Die Erfindung betrifft ein Desinfektionsmittel mit keimabtötenden Eigenschaften.

Die zunehmende Verbreitung von Mikroorganismen, wie Bakterien und Pilzen, sowie die steigende Bedrohung durch pathogene Viren (SARS, HIV, Grippe) in allen Bereichen unserer Gesellschaft macht eine nachhaltige Desinfektion notwendig. In den vergangenen Jahren entstanden aufgrund des zum Teil wahrlosen Einsatzes von Antibiotika immer mehr Mikroorganismen, die resistent gegen die verwendeten Chemotherapeutika sind. Anfänglich war das Vorkommen auf Kliniken beschränkt. Hiervon sind besonders die Einrichtungen und die Geräte (Monitore, Tastaturen) in Intensivstationen betroffen. Mittlerweile werden diese Problemkeime auch in Altenheimen und privaten Haushalten nachgewiesen.

Derzeit versucht man diesem Problem durch eine verstärkte Reinigung und Desinfektion zu begegnen. Vorteil der Desinfektion ist, dass nahezu alle Krankheitserreger innerhalb kürzester Zeit abgetötet werden. Nachteil ist, dass nach der Verdunstung des Desinfektionsmittels Keime sofort wieder die Oberfläche besiedeln und sich vermehren.

Ein großes Problem bei der Reinigung von Kliniken, Altenheimen und öffentlichen Einrichtungen ist außerdem, dass aus Kostengründen die Reinigungszeiten immer mehr verkürzt werden und damit keine ausreichende Reinigung stattfinden kann. Diese Verunreinigungen dienen Mikroorganismen als Basis zum Wachsen und verstärken das Problem eher noch.

Die derzeit eingesetzten Desinfektionsmittel basieren in der Regel auf der Kombination von Wasser, einem oder mehreren Alkoholen (z.B. Ethanol und/oder Isopropanol) und anderen Wirkstoffen, wie zum Beispiel Benzalkoniumchlorid, Formaldehyd, Tensiden und vielen anderen mehr. Definitionsgemäß müssen diese Desinfektionsmittel Mikroorganismen und Viren innerhalb einer vorgegeben Zeit abtöten. Entsprechende Vorschriften und Richtlinie werden von der Deutsche Gesellschaft für Hygiene und Mikrobiologie (DGHM; Richtlinien für die Prüfung chemischer Desinfektionsmittel) erarbeitet und im Rahmen einer Zulassung überprüft.

Das Problem besteht darin, dass zwar die Keime gemäß der Vorgabe der DGHM abgetötet werden, sich aber keine nachhaltige Wirksamkeit einstellt, d.h. nach kürzester Zeit wieder eine Besiedlung der frisch desinfizierten Oberfläche stattfinden kann. Zusätzlich erreicht man durch die kommerziell verwendeten Desinfektionsmittel keine Versiegelung der Oberfläche derart, dass die behandelte Oberfläche sich durch eine leichtere Reinigungsfähigkeit auszeichnet.

Aus der DE 199 35 230 C2 ist ein biofilmhemmendes Mittel bekannt, welches zur Beschichtung von Materialoberflächen von Nutzwasseranlagen und -behältnissen, zur algenabweisenden Beschichtung von Wasserfahrzeugen und maritimen Anlagen, zur antimikrobiellen Ausrüstung medizinischer, pharmazeutischer und biotechnischer Geräte und Gegenständen sowie zur antimikrobiellen Imprägnierung von Textilien verwendet werden kann. Diese Beschichtung genügt jedoch nicht den Ansprüchen, die an ein Desinfektionsmittel gestellt werden.

Die WO 2006/010667 A beschreibt Zusammensetzungen zur Behandlung von Holz, welche eine anhaltend biozide Wirkung aufweisen und bis zu 6% Alkylsilane, wie z.B. Methyltriethoxysilan, oder deren Hydrolyseprodukte umfassen können.

Die US 2003/118624 A1 beschreibt Zusammensetzungen welche Prekursoren, wie z.B. Tetraethoxysilan, und einen bioziden Stoff in alkoholischer Lösung umfassen, die, nachdem sie auf eine zu behandelnde Oberfläche aufgetragen wurden, bei erhöhten Temperaturen ausgehärtet werden.

Aufgabe der Erfindung ist es somit, ein neues Desinfektionsmittel mit akut und nachhaltig keimabtötenden Eigenschaften zu schaffen.

Diese Aufgabe wird erfindungsgemäß durch den gegenstand von Anspruch 1 gelöst.

Es konnte im Rahmen der Erfindung einerseits gezeigt werden, dass sich mit einem erfindungsgemäßen Desinfektionsmittel unterschiedliche Oberflächen, die in den oben angeführten Bereichen Anwendung finden (z.B. Metall, Keramik, Textilien, Kunststoffe), behandeln lassen. Durch die Mittel zum Ausbilden einer desinfizierenden und/oder nachhaltig antimikrobiell wirkende Komponenten freisetzenden Matrix wurden drei Ziele verwirklicht: Erstens werden alle Keime im Sinne eines Desinfektionsmittels sofort abgetötet. Zweitens etabliert sich auf der Oberfläche eine leicht zu reinigende Oberfläche, von der Verschmutzungen leichter zu entfernen sind. Drittens entwickelt sich gleichzeitig eine biozide Oberfläche, die über einen prolongierten Zeitraum Keime und Viren abtötet.

Neben der direkten keim- und virusabtötenden Wirkung stellen sich somit bei dem erfindungsgemäßen Desinfektionsmittel zwei weitere Wirkungen ein:
1. Es bildet sich nach dem Verdunsten des Lösungsmittels ein "Nano-Schwamm" aus, in dem Benzalkoniumchlorid eingelagert ist, das bei einer Kontaminierung/Besiedelung an die Oberfläche diffundiert und Bakterien, Viren und Pilze abtötet. Diese Wirkung wurde bis zu einer Dauer von 10 Tagen nachgewiesen und dürfte auch noch länger anhalten.
2. Der "Nano-Schwamm" etabliert zusätzlich eine leicht zu reinigende Oberfläche ("Easy-to-clean-Oberfläche"), welche den Reinigungsaufwand um bis zu 50% reduziert.

Untersucht wurde die Wirksamkeit beispielsweise anhand der Keime Escherichia coli, Pseudomonas aeruginosa, Staphylococcus aureus, Candida albicans und Aspergillus niger.

Einer der Vorteile der Erfindung besteht darin, dass keine Aktivierungsenergie in Form von Wärme oder UV-Strahlung erforderlich ist.

Erfindungsgemäß ist vorgesehen, dass das Desinfektionsmittel sowohl in der Anfangsphase Eigenschaften eines Desinfektionsmittels aufweist als auch die nachhaltig antimikrobiell wirkenden Komponenten über einen Zeitraum von Minuten, Stunden, Tagen oder Wochen aus der Matrix freisetzbar sind.

Gerade durch die langen Freisetzungszeiten wird eine nachhaltige Wirkung des Desinfektionsmittels erreicht.

Es liegt im Rahmen der Erfindung, dass das Desinfektionsmittel nach dem Auftragen des Desinfektionsmittels auf einen Gegenstand eine Oberfläche ausbildet, die eine verminderte Anhaftung von Biomolekülen, Mikroorganismen und Verschmutzungen aufweist und neben der nachhaltigen keimabtötenden Eigenschaft eine temporäre leicht zu reinigende Oberfläche ausbildet.

Der anorganisch-organische Netzwerkbildner Methyltriethoxysilan (MTEOS) dient als Mittel zum Ausbilden einer desinfizierenden und/oder nachhaltig Benzalkoniumchlorid freisetzenden Matrix während oder nach der Aufbringung des Desinfektionsmittels.

Die Verwendung des erfindungsgemäßen Desinfektionsmittels in Kliniken, Arztpraxen, Alten- und Pflegeheimen, Haushalten (z.B. Küche, Bad, WC), gewerblichen (z.B. Hotels, Gastronomie), militärischen oder industriellen Einrichtungen sowie auf folgenden Einrichtungen und Gegenständen: Tastaturen, Saunen, Solarien, Schwimmbäder, Automobile, Geräte und Apparaturen aus den Bereichen Medizin, Medizintechnik, Pharma, Kosmetik, Lebensmittel und Life Science liegt im Rahmen der Erfindung.

Die Lösung der oben genannten Probleme wird wie folgt verwirklicht:
Durch die Kombination Benzalkoniumchlorid mit MTEOS lässt sich eine initiale Abtötung an der Oberfläche befindlicher Keime erreichen. Zusätzlich wurde dadurch erwartungsgemäß eine wasser- und somit auch schmutzabweisende Versiegelung erreicht, welche die nachfolgenden Reinigungsschritte erleichtert. Die hydrophobe Versiegelung wurde durch die Zuhilfenahme der Sol-Gel-Chemie erreicht, wobei die silanbasierten Polykondensate das chemische Rückgrat für das Benzalkoniumchlorid ausbilden.

Erstaunlicherweise hat sich allerdings gezeigt, dass diese anorganisch-organischen Polykondensate in der Lage sind, das eingesetzte Desinfektionsmittel soweit zu verkapseln und einzubinden, dass sich ein Langzeit-Effekt ergibt, der ausschließlich durch eine kontrollierte und zeitverzögerte Abgabe des Desinfektionsmittels zu erklären ist.

Da es sich bei dem erfindungsgemäßen Desinfektionsmittel um ein Desinfektionsmittelanalogon handelt, war bei dieser Entwicklung die Tatsache essentiell, dass es sich um ein Mittel handelt, welches den Effekt bereits bei Raumtemperatur ausbildet.

Basierend auf dem oben Dargestellten wurden Mittel entwickelt, welche einerseits eine desinfizierende Wirkung beinhalten, gleichzeitig auch eine mindestens mehrere Tage andauernde (also nachhaltige) antimikrobielle Eigenschaft aufweisen und zugleich die Oberfläche so hydrophobieren, dass sich beispielsweise gegen Wasser Kontaktwinkel von größer 85° ausbilden, was einer Easy-to-Clean-Eigenschaft gleichkommt.

Die antimikrobiellen Eigenschaften wurden bereits anhand der Absterberate von Aspergillus niger, Staphylococcus aureus, Escherichia coli und Pseudomonas aeruginosa überprüft.

Ein Desinfektionsmittel, welches nicht erfindungsgemäß ist und das pro 100 g Lösung 49,4 g Ethanol, 7,1 g Isopropanol, 0,75 g Benzalkoniumchlorid (95 %) und 0,125 g Natriumpyrithion (40 %) als Wirkstoffe enthielt, wurde durch Mikrolab, Bremen (von der DVV (Deutsche Virologische Vereinigung, zugelassenes Prüflabor)) auf seine antivirale Wirksamkeit mit dem Ergebnis "bedingte Viruzidie" überprüft.

Die Biokompatibilität dieses Desinfektionsmittels wurde durch Bioservice Scientific Laboratories GmbH unter GLP-Bedingungen nachgewiesen.

Zudem wurde festgestellt, daß dieses Desinfektionsmittel aufgrund der keimabtötenden Wirkung die bakteriell verursachte Ausbildung von geruchsintensiven Stoffen, wie z.B. Ammoniak (Sanitärbereich), deutlich reduziert.

Dieses Desinfektionsmittel zeigte bei einer Untersuchung durch das SGS Institut Fresenius GmbH, Taunusstein, in Anlehnung an ASTM E 2180 folgende Ergebnisse (im Vergleich zu einer unbehandelten Keramiktestfläche):

| Zeit zwischen Auftragen und ASTM-Test (Δt) | 3 Tage | | 5 Tage | | 10 Tage | |
|---|---|---|---|---|---|---|
| Reduktion nach | 0,5h | 24 h | 0,5 h | 24 h | 0,5 h | 24 h |
| Escherichia coli | > 99,997 % | > 99,997 % | 99,694 % | > 99,994 % | 97,609 % | > 99,996 % |
| Pseudomonas aeruginosa | >99,996 % | > 99,996 % | 99,968 % | > 99,995 % | 95,294 % | > 99,997 % |
| Staphylococcus aureus | > 99,997 % | > 99,997 % | > 99,996 % | > 99,996 % | > 94,545 % | > 99,995 % |
| Candida albicans | 99,636 % | > 99,997 % | -(k.R) ¹ | > 99,992 % | -(k.R) ¹ | > 99,997 % |
| Aspergillus niger | 99,557 % | > 99,995 % | 8,333 % | > 99,992 % | 98,628 % | > 99,837 % |

| | | | | | | |
|---|---|---|---|---|---|---|
| ¹ keine Reduktion (in Anlehnung an den ASTM E 2180, getestet von SGS Institut Fresenius) | | | | | | |

Die antimikrobielle Wirkung wurde zu den Zeitpunkten 3, 5 und 10 Tage wie folgt bestätigt:

### 3 Tage:

Starke bakterizide Wirkung gegenüber Escherichia coli, Pseudomonas aeruginosa und Staphylococcus aureus, stark fungizide Wirkung gegenüber Aspergillus niger und Candida albicans.

### 5 Tage:

Starke bakterizide Wirkung gegenüber Escherichia coli, Pseudomonas aeruginosa und Staphylococcus aureus, stark fungizide Wirkung gegenüber Aspergillus niger und Candida albicans.

### 10 Tage:

Starke bakterizide Wirkung gegenüber Escherichia coli, Pseudomonas aeruginosa und Staphylococcus aureus, stark fungizide Wirkung gegenüber Candida albicans, signifikant fungizide Wirkung gegenüber Aspergillus niger.

Aufgrund seiner langzeitantimikrobiellen Eigenschaften wurde das Desinfektionsmittel durch die Desinfektionsmittel-Kommission im VAH (Verbund für Angewandte Hygiene) in die Desinfektionsmittelliste der DGHM aufgenommen.

Auch der Easy-to-clean-Effekt wurde von SGS Institut Fresenius GmbH, Taunusstein geprüft und bestätigt mit dem Ergebnis: Verringerung des Reinungsaufwandes einer behandelten Oberfläche um 50 %.

## Patentansprüche

1. Desinfektionsmittel herstellbar dadurch, dass zu einem Lösungsmittelgemisch aus Wasser, Isopropanol und Ethanol im Verhältnis 20/70/10 Benzalkoniumchlorid gegeben wird, so dass sich eine 2-prozentige Lösung ergibt und 1 bis 100 g/l, bezogen auf das Lösungsmittelgemisch, des Vorhydrolysates des anorganischorganischen Netzwerkbildners Methyltriethoxysilan zugegeben werden.

2. Desinfektionsmittel gemäß Anspruch 1, **dadurch gekennzeichnet, dass** 5 bis 50 g/l, bezogen auf das Lösungsmittelgemisch, des Vorhydrolysates des anorganisch-organischen Netzwerkbildners Methyltriethoxysilan zugegeben werden.

## Claims

1. Disinfectant that can be produced by adding benzalkonium chloride to a solvent mixture consisting of water, isopropanol and ethanol in a ratio of 20/70/10 in such manner that a 2 per cent solution is obtained, and adding 1 to 100 g/l, relative to the solvent mixture, of the pre-hydrolysate of the inorganic/organic network former methyltriethoxysilane.

2. Disinfectant according to claim 1, **characterised in that** 5 to 50 g/l, relative to the solvent mixture, of the pre-hydrolysate of the inorganic/organic network former methyltriethoxysilane are added.

## Revendications

1. Agent de désinfection pouvant être fabriqué en ce que du chlorure de benzalkonium est ajouté à un mélange de solvant consistant en de l'eau, de l'isopropanol et de l'éthanol dans un rapport de 20/70/10, de sorte à obtenir une solution à 2 %, et 1 à 100 g/l, rapporté au mélange de solvant, du préhydrolysat de l'agent de réticulation minéralo-organique methyltriéthoxysilane sont ajoutés.

2. Agent de désinfection selon la revendication 1, **caractérisé en ce que** 5 à 50 g/l, rapporté au mélange de solvant, du préhydrolysat de l'agent de réticulation minéralo-organique méthyltriéthoxysilane sont ajoutés.
